# EUROPEAN PATENT APPLICATION

(11) **EP 3 943 934 A1**
(43) Date of publication of application: **26.01.2022**
(21) Application number: 20783522.4
(22) Date of filing: 26.03.2020
(51) Int. Cl.: G01N 33/49

(54) **LIQUID PATH SYSTEM AND CONTROL METHOD THEREFOR**

(30) Priority: 29.03.2019 CN 201910252762
(71) Applicant: Hemoassay Science and Technology (Suzhou) Co., Ltd., Suzhou, Jiangsu 215000 (CN)
(72) Inventor: XIAO, Jian, Suzhou, Jiangsu 215000 (CN); HU, Xi, Suzhou, Jiangsu 215000 (CN); CHEN, Jienian, Suzhou, Jiangsu 215000 (CN); WANG, Tao, Suzhou, Jiangsu 215000 (CN); JIANG, Feng, Suzhou, Jiangsu 215000 (CN)
(74) Representative: Wang, Bo
(86) International application number: PCT/CN2020/081461
(87) International publication number: WO 2020/200048

(57) **Abstract**

The invention discloses a fluid path system and a control method thereof. Said system includes a blood sample fluid path system and a reagent fluid path system, wherein the blood sample fluid path system includes a blood-suctioning member, a first suction-controlling member, and a suctioning member-cleaning device, the first suction-controlling member leads to the blood-suctioning member, so as to control the blood-suctioning member to suction and release blood, the suctioning member-cleaning device includes a first fluid path valve connected with the first suction-controlling member, and the first fluid path valve leads to a gas source and a water source, the reagent fluid path system includes at least one reagent-suctioning member and a second suction-controlling member connected to the reagent-suctioning member. The invention achieves completely automatically adding blood and reagents during detecting blood coagulation data.

## Description

### TECHNICAL FIELD OF THE INVENTION

The invention relates to a thromboelastometer, in particular to a fluid path system and a control method thereof of the thromboelastometer.

### DESCRIPTION OF THE PRIOR ART

A thromboelastometer is a medical device used to test whether blood can coagulate normally. It is generally necessary to detect the patient's blood coagulation data through the thrombo elastometer before performing an operation, and judge whether the patient's blood coagulation process is normal based on the detected blood coagulation data. Only when the blood is able to coagulate normally, the patient can be operated on. If the blood coagulation process is abnormal, the patient's blood will not be able to coagulate normally during the operation, leading to difficulty in hemostasis, which may endanger the patient's life.

The physical properties of testing a blood clot are based on the following principles. A special cylindrical cup statically containing blood rotates at a low angular speed of 4.75, and a testing rod immersed in the blood sample is used to detect the movement of the blood sample. After the cup adheres to the testing rod through a fibrin platelet complex, the rotational force generated by rotating the cup can be transmitted to the testing rod in the blood sample. The strength of the fibrin platelet complex can influence the movement amplitude of the testing rod, so that a strong blood clot can synchronize the movements of both the testing rod and the cup. Therefore, the movement amplitude of the testing rod is directly related to the strength of the formed blood clot. In the case that the blood clot retracts or dissolves, the joint between the testing rod and the blood clot is released, so the movement of the cup is no longer transmitted to the testing rod, the rotation of which is detected by the sensor.

As for the current thromboelastometer, when detecting blood coagulation data, it is necessary to manually add blood and reagents to a testing cup, which requires a lot of time for operators, resulting in high labor intensity and low test efficiency for detecting blood coagulation data. In addition, manual addition depends greatly on the technical level of operators, which affects test accuracy.

### SUMMARY OF THE INVENTION

A objective of the invention is to overcome the defects of the prior art and provide a fluid path system and a control method thereof that achieve automatically adding blood and reagents.

To achieve the above object, the present invention provides the following technical solutions: a fluid path system, the device comprises a blood sample fluid path system and a reagent fluid path system, wherein the blood sample fluid path system includes a blood-suctioning member, a first suction-controlling member, and a suctioning member-cleaning device, the first suction-controlling member leads to the blood-suctioning member, so as to control the blood-suctioning member to suction and release blood, the suctioning member-cleaning device includes a first fluid path valve connected with the first suction-controlling member, and the first fluid path valve leads to a gas source and a water source, the reagent fluid path system includes at least one reagent-suctioning member and a second suction-controlling member connected to the reagent-suctioning member.

Preferably, the blood sample fluid path system further includes a suctioning-member outer-wall-cleaning device, the suctioning-member outer-wall-cleaning device includes a suctioning member-cleaning trough connected to a water source, the blood-suctioning member extends into the suctioning member-cleaning trough, a water source inlet connected to the water source is provided on the side wall of the suctioning member-cleaning trough, the water source inlet is located above the blood sample on the outer wall of the blood-suctioning member.

Preferably, the suctioning member-cleaning device further includes a check valve, both the ends of the check valve lead to the first suction-controlling member and the first fluid path valve, respectively.

Preferably, the suctioning member-cleaning device further includes a first diaphragm pump connected to the check valve and the first fluid path valve.

Preferably, the first suction-controlling member is a plunger pump, and the first fluid path valve is a three-way fluid path valve.

Preferably, a second diaphragm pump is further connected between the water source inlet and the water source.

Preferably, the blood sample fluid path system may further include a sewage-collecting device, the sewage-collecting device includes a third diaphragm pump and a sewage barrel connected to the third diaphragm pump, the third diaphragm pump is connected with the sewage outlet installed on the bottom of the suctioning member-cleaning trough.

This present invention also discloses another technical solution: a fluid path control method, comprises a blood sample fluid path control method and a reagent fluid path control method,
wherein the blood sample fluid path control method includes the following steps:
   A1, the first suction-controlling member controlling the blood-suctioning member to suction blood from a blood sample and release it into a testing cup,
   A2, closing the first suction-controlling member, opening the first fluid path valve, controlling the water source to enter the blood-suction member for cleaning the latter,
   A3, after cleaning, making a switch to control air source to blow into the blood-suctioning member and to blow off the water source remaining in the blood-suctioning member.
the reagent fluid path control method includes the following steps,
   B1, the second suction-controlling member controlling the reagent-suctioning member to suction a reagent from the reagents corresponding to the reagent-suctioning member and releasing it into a testing cup.

Preferably, the blood sample fluid path control method further includes:
A4, cleaning the outer wall of the blood-suctioning member.

Preferably, the A4 specifically includes, extending the blood-suctioning member into the suctioning member-cleaning trough, and the water source entering the suctioning member-cleaning trough through the water source inlet to clean the outer wall of the blood-suctioning member.

Preferably, the blood sample fluid path control method further includes:
A5, collecting the sewage after washing the blood-suctioning member.

Preferably, the A5 specifically includes, the third diaphragm pump controlling the sewage to enter the sewage barrel from the sewage outlet at the bottom of the suctioning member-cleaning trough and to be collected.

The beneficial effects of the present invention are as follows.
1. The invention achieves completely automatically adding blood and reagents during detecting blood coagulation data through the designed fluid path system, and replacing manual operation, which reduces the labor intensity of detecting blood coagulation data, and reduces the production cost. In addition, the fluid path system runs reliably, and has good repeatability, and a low error rate, which improves test accuracy.
2. The blood sample fluid path is cleaned by matching a water source with aair source, so that the inside of the blood-suctioning member is thoroughly cleaned, in addition, while the inner wall of the blood-suctioning member is cleaned, the outer wall of the blood-suctioning member is also cleaned, which further improves the cleanliness of the blood-suctioning member to avoid cross-contamination of different blood samples.
3. It recycles the sewage after cleaning, so it has energy saving and environmental protection.

### BRIEF DESCRIPTION OF THE DRAWINGS

FIG.1 is a block diagram of the fluid path system according to the invention.
FIGs.2,5-10 are all a block diagram for the different embodiments of the blood sample fluid path system according to the invention.
FIG.3 is a schematic diagram of the blood-suctioning member of the invention.
FIG.4 is a schematic diagram of the first/second suction-controlling member of the invention.
FIG. 11 is a block diagram of the reagent fluid path system according to the invention.
FIG. 12 is a schematic diagram of the reagent-suctioning member of the invention.
FIGs. 13-14 are a flow chart for the different embodiments of the fluid path control method according to the invention.
FIG. 15 is a specific structure schematic diagram of the blood sample fluid path system corresponding to the embodiment of FIG. 10.
FIG. 16 is a specific structure schematic diagram of the reagent fluid path system corresponding to the embodiment of FIG. 11.

Wherein:
1- fluid path system; 2- blood sample fluid path system; 21- blood-suctioning member; 22- first suction-controlling member; 23- suctioning member-cleaning device; 231- first fluid path valve; 232- check valve; 233- first diaphragm pump; 24- suctioning-member outer-wall-cleaning device; 241- second diaphragm pump; 2411- water source inlet; 2412-sewage outlet; 242- second diaphragm pump; 25- sewage-collecting device; 251- third diaphragm pump; 252- sewage barrel; 3- reagent fluid path system; 31- reagent-suctioning member; 32- second suction-controlling member; 33- second fluid path valve; 4- water tank; 5- air source; 6- integrated rod; 61- connecting port; 7- connector.

### DETAILED DESCRIPTION OF THE INVENTION

The technical solutions of the embodiments in the invention will be clearly and completely described below in combination with the figures in the invention.

A fluid path system and a control method thereof disclosed by the invention achieves automatically adding blood and reagents during detecting blood coagulation data.

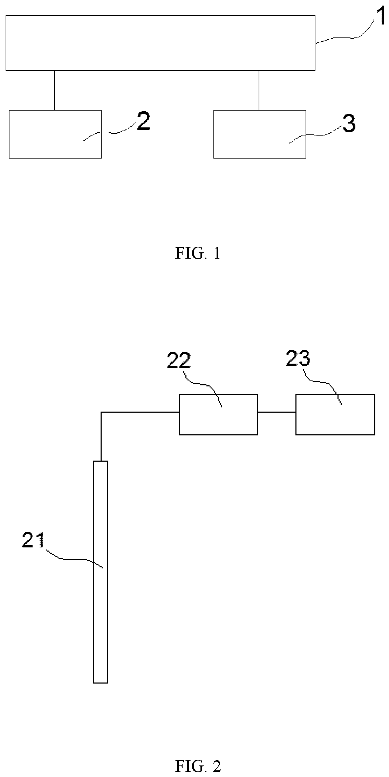

As shown in FIG. 1, a fluid path system 1 disclosed in an embodiment according to the invention includes an independent blood sample fluid path system 2 and an independent reagent fluid path system 3, wherein the blood sample fluid path system 2 is used for automatically adding blood during detecting blood coagulation data, the reagent fluid path system 3 is used for automatically adding reagents during detecting blood coagulation data.

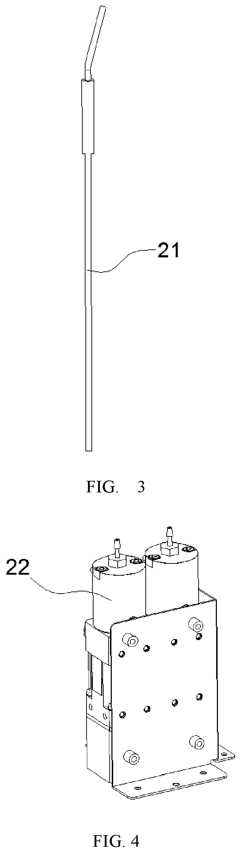

Specifically, in this embodiment, as shown in FIG. 2, the blood sample fluid path system 2 includes a blood-suctioning member 21, a first suction-controlling member 22, and a suctioning member-cleaning device 23, wherein the blood-suctioning member 21 is used for suctioning blood, as shown in FIG.3, it has a hollow syringe needle-like shape as a whole, when suctioning blood, its lower end goes deep into the blood sample testing tube (not shown in the figures).

The first suction-controlling member 22 leads to the blood-suctioning member 21, specifically connected to the upper end of the blood-suctioning member 21 through a hose (such as a PVC tube), when implemented, as shown in FIG. 4, the first suction-controlling member 22 can be a plunger pump. The working principle is that when the first suction-controlling member 22 operates, it controls the blood-suctioning member 21 to form negative pressure, and to suction the blood from a blood sample testing tube, when the first suction-controlling member 22 stops, it controls the blood-suctioning member 21 to release the suctioned blood into a testing cup (not shown in the figures).

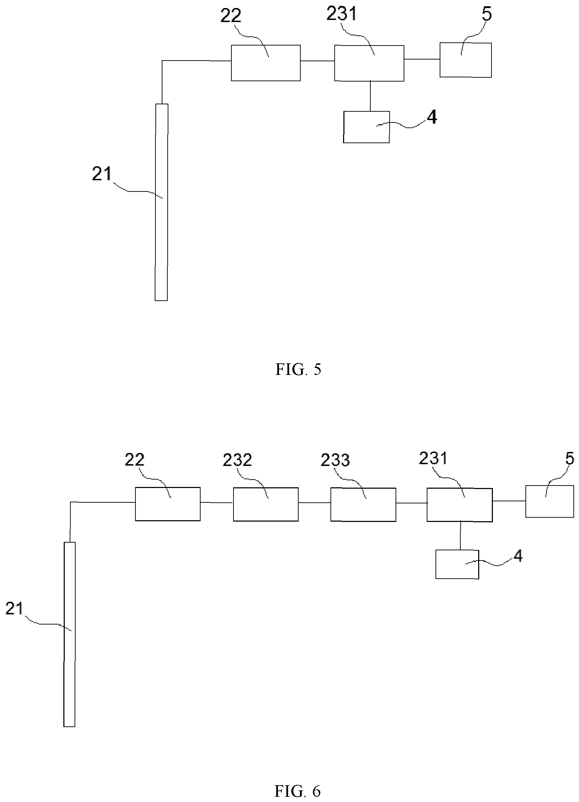

The suctioning member-cleaning device 23 is connected to the blood-suctioning member 21, used for thoroughly cleaning the inner wall of the blood-suctioning member 21 after the blood-suctioning member 21 releases blood into the testing cup. Specifically, as shown in FIG. 5, in this embodiment, the suctioning member-cleaning device 23 includes a first fluid path valve 231, wherein the first fluid path valve 231 leads to the blood-suctioning member 21 through the first suction-controlling member 22, and the first fluid path valve 231 further leads to a gas source and a water source. When implemented, the first fluid path valve 231 can be a control valve with at least three joint openings, such as a two-position three-way fluid path valve, and of course, it can also be a four-way valve, a five-way valve, and the like. One of the joint openings of the three-way fluid path valve leads to the first suction-controlling member 22, and the other two joint openings are connected to the air source 5 and the water source 4, respectively. When implemented, the water source 4 can be clean water, and the clean water is stored in a water tank, that is, the three-way fluid path valve is connected to the water tank, the air source 5 can be air. In addition, the first fluid path valve 231 and the water source 4 can also be connected with each other through a connector 7.

When cleaning, firstly the three-way fluid path valve 231 controls the joint opening connected to the water tank 4 to be in an on state, and the joint opening connected to the air source 5 to be in an off state, after that, the first suction-controlling member 22 controls it to make the water source suctioned from the water tank 4 entry the blood-suctioning member 21 through each connecting hose, after washing, in order to blow off the cleaning liquid remaining on the inner wall of the blood-suctioning member 21, the three-way fluid path valve 231 controls the joint opening connected to the water tank 4 to be in an off state, and the joint opening connected to the air source 5 to be in an on state, after that, the first suction-controlling member 22 controls it to make the air source 5 suctioned into the blood-suctioning member 21 through each connecting hose, so as to blow off the cleaning liquid remaining on the inner wall of the blood-suctioning member 21. In this way, the blood-suctioning member 21 is deeply cleaned and blown dry, so that it can suction other types of blood samples and avoid cross-contamination between different blood samples.

Preferably, as shown in FIG. 6, in order to prevent blood from flowing into the water tank 4 or the air source 5 through the first suction-controlling member 22, or to avoid the backflow of the water source 4 and the air source 5, a check valve 232 can be addedly provided between the first fluid path valve 231 and the first suction-controlling member 22, that is, restricts the water source 4 and the air source 5 to unidirectional flow into the blood-suctioning member 21, if reversed flow, the passage is disconnected . The check valve 232 is closed when the first suction-controlling member 22 is started to control the blood-suctioning member 21 to suction blood, and is opened when the suctioning member-cleaning device 23 is operating.

More preferably, as shown in FIG. 6, a first diaphragm pump 233 is connected between the check valve 232 and the first fluid path valve 231, used for matching with the first suction-controlling member 22 to achieve suction control to the water source 4 and the air source 5.

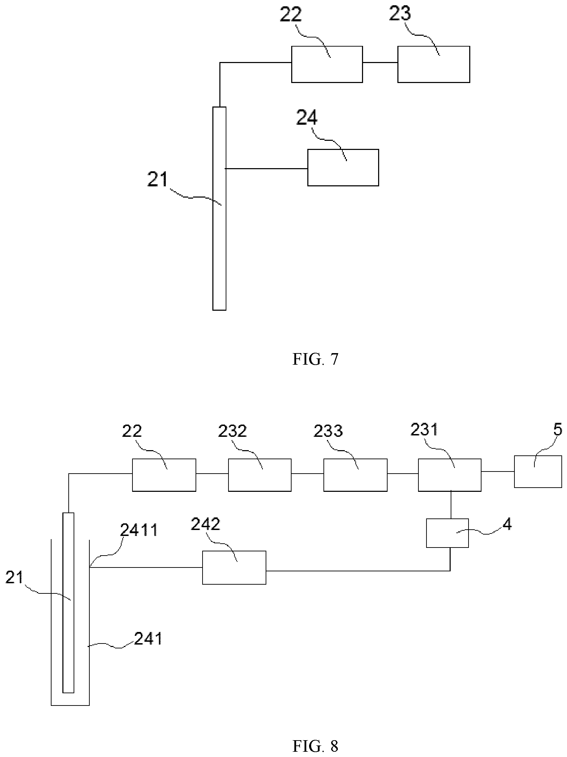

Further, in order to clean the outer wall of the blood-suctioning member while cleaning the inner wall of the blood-suctioning member, as shown in FIG. 7, the blood sample fluid path system 2 of the embodiment according to the invention further includes a suctioning-member outer-wall-cleaning device 24, which specifically includes a suctioning member-cleaning trough 241 and a second diaphragm pump 242, wherein the blood-suctioning member 21 extends into the suctioning member-cleaning trough 241, and the suctioning member-cleaning trough 241 is connected to the above-mentioned water source (ie, the water tank 4) through the second diaphragm pump 242, that is, the second diaphragm pump 242 is connected with the suctioning member-cleaning trough 241 and the water tank 4, used for controlling the water in the water tank 4 to be suctioned into the suctioning member-cleaning trough 241, so as to clean the outer wall of the blood-suctioning member 21 inside the suctioning member-cleaning trough 241.

Specifically, as shown in FIG. 8, a water source inlet 2411 is provided on the side wall of the suctioning member-cleaning trough 241 close to its top, and the water source inlet 2411 leads to the second diaphragm pump 242. Preferably, in order to ensure that the blood contaminated on the outer wall of the blood-suctioning member 21 can be cleaned when the blood is suctioned, in the embodiment according to the invention, the height of the water source inlet 2411 in the suctioning member-cleaning trough 241 is set to be higher than the height of the blood sample on the outer wall of the blood-suctioning member 21 extending into the suctioning member-cleaning trough 241, that is, above the blood sample on the outer wall of the blood-suctioning member 21, so as to ensure that the water entering the blood-suctioning member 21 can completely cover and wash the blood sample on the outer wall of the blood-suctioning member 21 to make the blood sample on the outer wall of the blood-suctioning member 21 cleaned thoroughly.

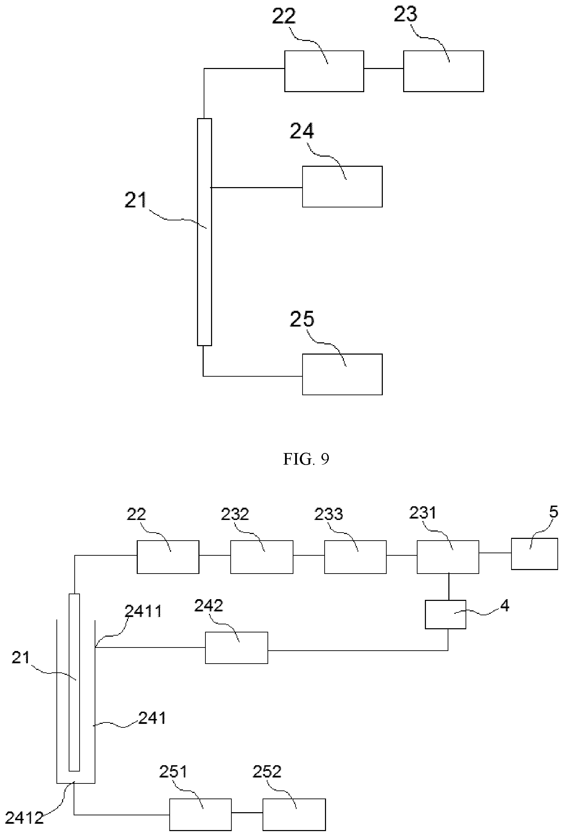

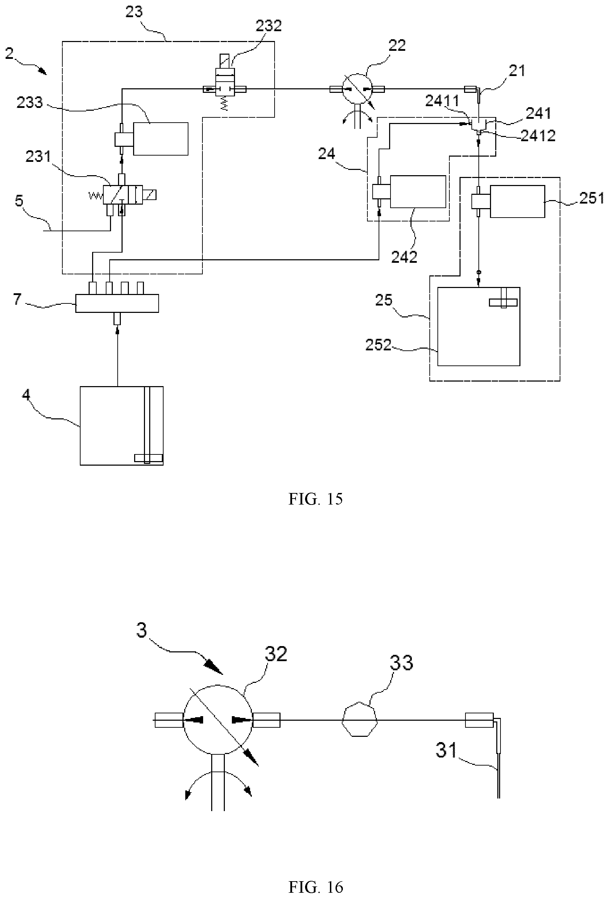

Further, as shown in FIG. 9, the blood sample fluid path system in the embodiment according to the invention may further include a sewage-collecting device 25, which is connected to the suctioning member-cleaning trough 241, and is used to collect and discharge the sewage generated after cleaning the suctioning member-cleaning device 23 and the suctioning-member outer-wall-cleaning device 24. Specifically, as shown in FIG. 10 and FIG. 15, in this embodiment, the sewage-collecting device 25 includes a third diaphragm pump 251 and a sewage barrel 252 connected to the third diaphragm pump 251, wherein a sewage outlet 2412 is provided on the suctioning member-cleaning trough 241, specifically is arranged at the bottom. The third diaphragm pump 251 is connected with the sewage outlet 2412 and the sewage barrel 252, and is used to control the sewage generated in the suctioning member-cleaning trough 241 after cleaning to be collected and discharged into the sewage barrel 252.

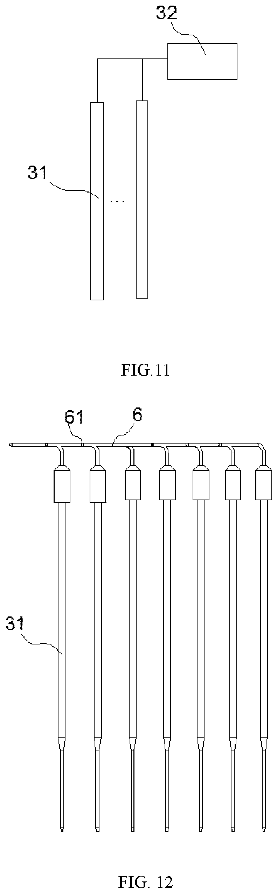

In this embodiment, as shown in FIG. 11 and FIG. 16, the reagent fluid path system 3 specifically includes at least one reagent-suctioning member 31 and a second suction-controlling member 32 connected to the reagent-suctioning member 31, wherein the reagent-suctioning member 31 is used to suction the reagents required for the test, when implemented, the reagent-suctioning member 31 is generally set to be several ones, each of which suctions one kind of reagents, in this embodiment, as shown in FIG.12, seven reagent-suctioning members 31 are provided, that is, suction 7 kinds of reagents correspondingly, of course, the number of reagent-suctioning members 31 is not limited and can be set according to the requirements for the reagents. The second fluid path valve 33 controls which reagent to be taken, and is arranged between the reagent-suctioning member 31 and the second suction-controlling member 32. Each reagent-suctioning member 31 also has a hollow syringe needle-like shape as a whole, when suctioning reagents, its lower end goes deep into the reagent testing tube (not shown in the figures). In addition, 7 reagent-suctioning members 31 are all connected to an integrated rod 6, and a connecting port 61 is provided on the integrated rod 6 at a position corresponding to each reagent-suctioning member 31.

The second suction-controlling member 32 is selectively connected to the connecting port 61 on the integrated rod 6 as required to realize the connection with the reagent-suctioning member 31 corresponding to the connecting port 61. The second suction-controlling member 32 may be a plunger pump. Since each reagent-suctioning member 31 suctions one kind of reagents correspondingly, there is no cross-contamination between each other, so the reagent fluid path system here may not be provided with a corresponding cleaning device.

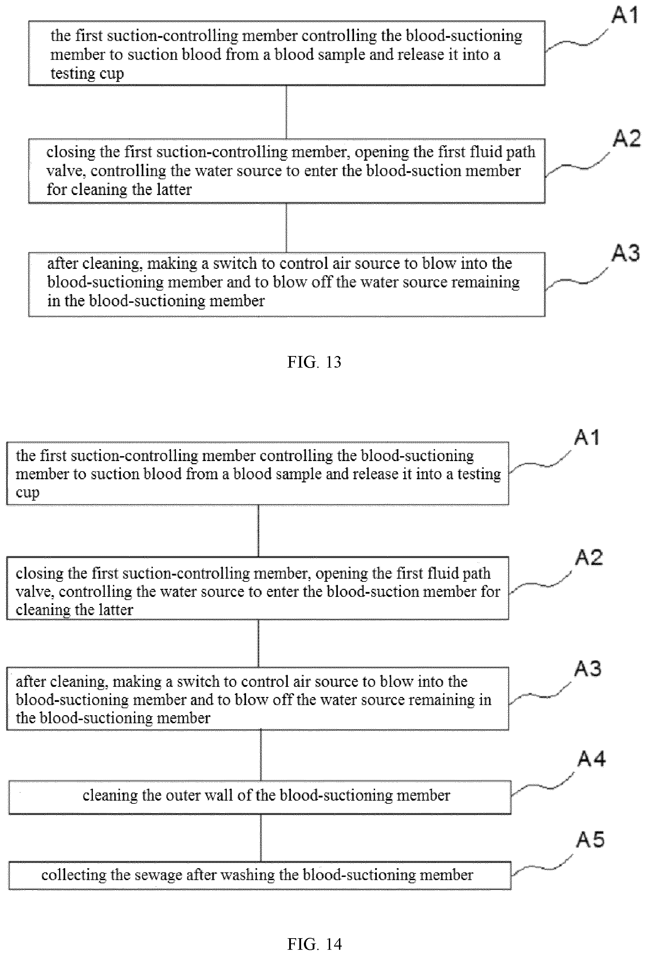

Based on the above fluid path system, as shown in FIG. 13, the embodiment according to the invention also discloses a fluid path control method, including a blood sample fluid path control method and a reagent fluid path control method, wherein the blood sample fluid path control method specifically includes the following steps:
A1 The first suction-controlling member 22 controlling the blood-suctioning member 21 to suction blood from a blood sample and release it into a testing cup.

Specifically, when the first suction-controlling member 22 operates, it controls the blood-suctioning member 21 to form negative pressure, and to suction the blood from a blood sample tube, when the first suction-controlling member 22 stops, it controls the blood-suctioning member 21 to release the suctioned blood into the testing cup.

A2 Opening the first fluid path valve 231, and the first suction-controlling member 22 controlling the water source to enter the blood-suction member 21 for cleaning the latter.

Specifically, the check valve 232 is opened, and the first fluid path valve 231 controls the joint opening connected to the water tank 4 to open, the first suction-controlling member 22 matches with the first diaphragm pump 233 to draw water from the water tank 4 and sent it into the inside of the blood-suctioning member 21, so as to clean the inner wall of the blood-suctioning member 21.

A3 After cleaning, making a switch to control air source 5 to blow into the blood-suctioning member 21 and to blow off the water source remaining in the blood-suctioning member 21.

Specifically, after having cleaned the inner wall of the blood-suctioning member 21, the check valve 232 is still open, and the first fluid path valve 231 controls the joint opening connected to the air source 5 to open, the first suction-controlling member 22 matches with the first diaphragm pump 233 to draw air resource 5 and sent it into the inside of the blood-suctioning member 21, so as to blow off water resource remaining inside the blood-suctioning member 21.

As shown in FIG. 14, preferably, after step A3, it further includes A4 to clean the outer wall of the blood-suctioning member 21. Specifically, the second diaphragm pump 242 controls the water in the water tank 4 to be sucked into the suctioning member-cleaning trough 241, and to clean the outer wall of the blood-suctioning member 21 in the suctioning member-cleaning trough 241.

As shown in FIG. 14, more preferably, after step A3, it further includes A4 to collect the sewage after washing the blood-suctioning member 21. Specifically, the third diaphragm pump 251 controls the sewage to enter the sewage barrel 252 from the sewage outlet 2412 at the bottom of the suctioning member-cleaning trough 241 and to be collected.

The reagent fluid path control method includes the following steps,
B1 the second suction-controlling member 32 controlling the reagent-suctioning member 31 to suction a reagent from the reagents corresponding to the reagent-suctioning member 31 and releasing it into a testing cup.

Specifically, the second suction-controlling member 32 suctions the reagent as required in the corresponding reagent-suctioning member 31, and controls the reagent-suctioning member 31 to release the suctioned reagent into the testing cup.

The technical content and technical features of the invention have been disclosed as above, but a person skilled in the art may still make various replacements and modifications based on the teachings and disclosures of the invention without departing from the essence of the invention. Therefore, the protection scope of the invention should not be limited to the content disclosed in the embodiments, but should include various replacements and modifications that do not deviate from the invention, and are covered by the claims of this patent application.

## Claims

1. A fluid path system, **characterized in that** said device comprises a blood sample fluid path system and a reagent fluid path system, wherein said blood sample fluid path system includes a blood-suctioning member, a first suction-controlling member, and a suctioning member-cleaning device, said first suction-controlling member leads to said blood-suctioning member, so as to control said blood-suctioning member to suction and release blood, said suctioning member-cleaning device includes a first fluid path valve connected with said first suction-controlling member, and said first fluid path valve leads to a gas source and a water source, said reagent fluid path system includes at least one reagent-suctioning member and a second suction-controlling member connected to said reagent-suctioning member.

2. The fluid path system according to claim 1, **characterized in that** said blood sample fluid path system further includes a suctioning-member outer-wall-cleaning device, said suctioning-member outer-wall-cleaning device includes a suctioning member-cleaning trough connected to a water source, said blood-suctioning member extends into said suctioning member-cleaning trough, a water source inlet connected to said water source is provided on the side wall of said suctioning member-cleaning trough, said water source inlet is located above the blood sample on the outer wall of said blood-suctioning member.

3. The fluid path system according to claim 1, **characterized in that** said suctioning member-cleaning device further includes a check valve, both the ends of said check valve lead to the first suction-controlling member and the first fluid path valve, respectively.

4. The fluid path system according to claim 3, **characterized in that** said suctioning member-cleaning device further includes a first diaphragm pump connected to said check valve and said first fluid path valve.

5. The fluid path system according to claim 1, **characterized in that** said first suction-controlling member is a plunger pump, and said first fluid path valve is a three-way fluid path valve.

6. The fluid path system according to claim 2, **characterized in that** a second diaphragm pump is further connected between said water source inlet and said water source.

7. The fluid path system according to claim 2, **characterized in that** said blood sample fluid path system may further include a sewage-collecting device, said sewage-collecting device includes a third diaphragm pump and a sewage barrel connected to said third diaphragm pump, said third diaphragm pump is connected with the sewage outlet installed on the bottom of said suctioning member-cleaning trough.

8. A fluid path control method based on the above fluid path system according to any one of claims1-7, **characterized in that** said method comprises a blood sample fluid path control method and a reagent fluid path control method, wherein said blood sample fluid path control method includes the following steps,
A1, the first suction-controlling member controlling the blood-suctioning member to suction blood from a blood sample and release it into a testing cup,
A2, closing the first suction-controlling member, opening the first fluid path valve, controlling the water source to enter the blood-suction member for cleaning the latter,
A3, after cleaning, making a switch to control air source to blow into the blood-suctioning member and to blow off the water source remaining in the blood-suctioning member.
said reagent fluid path control method includes the following steps,
B1, the second suction-controlling member controlling the reagent-suctioning member to suction a reagent from the reagents corresponding to the reagent-suctioning member and releasing it into a testing cup.

9. The fluid path control method according to claim 8, **characterized in that** said blood sample fluid path control method further includes
A4, cleaning the outer wall of the blood-suctioning member.

10. The fluid path control method according to claim 8, **characterized in that** said blood sample fluid path control method further includes,
A5, collecting the sewage after washing the blood-suctioning member.
